(19) **Europäisches Patentamt**

**European Patent Office**

**Office européen des brevets**

(11) **EP 0 916 089 B1**

(12) **EUROPÄISCHE PATENTSCHRIFT**

(45) Veröffentlichungstag und Bekanntmachung des Hinweises auf die Patenterteilung:
**13.02.2002 Patentblatt 2002/07**

(51) Int Cl.7: **G01N 27/82**

(21) Anmeldenummer: **97935497.4**

(86) Internationale Anmeldenummer:
**PCT/DE97/01620**

(22) Anmeldetag: **31.07.1997**

(87) Internationale Veröffentlichungsnummer:
**WO 98/05952 (12.02.1998 Gazette 1998/06)**

(54) **VORRICHTUNG UND VERFAHREN ZUR UNTERDRÜCKUNG VON SIGNALEN DER QUERBÜGEL BEI DER UNTERSUCHUNG VON SPANN-BETONBAUTEILEN MIT DER METHODE DER MAGNETISCHEN RESTFELDMESSUNG**

PROCESS AND DEVICE FOR SUPPRESSING CROSS GIRDER SIGNALS WHEN INSPECTING PRESTRESSED CONCRETE CONSTRUCTION ELEMENTS BY THE RESIDUAL MAGNETIC FIELD MEASUREMENT METHOD

PROCEDE ET DISPOSITIF POUR SUPPRIMER LES SIGNAUX EMIS PAR L'ETRIER TRANSVERSAL LORS D'ESSAIS DE COMPOSANTS EN BETON PRECONTRAINT SELON LA METHODE DE LA MESURE DU CHAMP MAGNETIQUE RESIDUEL

(84) Benannte Vertragsstaaten:
**AT BE CH DE FR GB IT LI**

(30) Priorität: **03.08.1996 DE 19631490**

(43) Veröffentlichungstag der Anmeldung:
**19.05.1999 Patentblatt 1999/20**

(73) Patentinhaber:
• **FORSCHUNGSZENTRUM JÜLICH GMBH 52425 Jülich (DE)**
• **FORSCHUNGS- UND MATERIALPRÜFUNGSANSTALT BADEN-WÜRTTEMBERG D-70569 Stuttgart (DE)**

(72) Erfinder:
• **BOUSACK, Herbert D-52080 Aachen (DE)**
• **KRAUSE, Hans-Joachim D-52080 Aachen (DE)**
• **SAWADE, Gottfried D-70619 Stuttgart (DE)**

(74) Vertreter: **Möbus, Steffen Kayser & Möbus Patentanwälte Leienfelsstrasse 6 81243 München (DE)**

(56) Entgegenhaltungen:
DE-A- 4 037 992    US-A- 4 503 393
US-A- 4 573 013

**Beschreibung**

Die vorliegende Erfindung betrifft eine Prüfanordnung zur Untersuchung der Integrität von Bauteilen, wie z. B. der Spannbewehrung von Spannbetonbauteilen, mit einem Prüfkopf, der eine Magnetisierungseinrichtung zur Erzeugung eines das Bauteil umgebenden Magnetfeldes aufweist und mit einer Steuereinrichtung zur Steuerung der Magnetisierung und zur Verarbeitung der dem Magnetfeld entsprechenden Signale verbunden ist sowie ein Verfahren für eine solche Untersuchung.

Brüche der Spannbewehrung werden durch charakteristische Anomalien des das Bauteil umgebenden Magnetfeldes erkannt, die auf lokale Änderungen der Magnetisierung bzw. der magnetischen Permeabilität der Spannbewehrung beruhen. Die Messung des Magnetfeldes erfolgt während der Magnetsierung (Messung im aktiven Feld) bzw. nach dem Magnetisieren (Restfeldmessung). Die Schwierigkeit bei der Bewertung der magnetischen Streufeldsignale besteht darin, daß etwaige Bruchsignale durch die Signale der oberflächennahen schlaffen Bewehrung überlagert werden.

Seit einiger Zeit wird zur nichtinvasiven Untersuchung der Integrität der Spannbewehrung von Spannbetonbauteilen die Prüfanordnung und Methode der magnetischen Streufeldmessung eingesetzt. In erster Linie werden die Streufeldsignale durch die Querbügel beeinflußt. Da die Prüfung des Bauteils über einen Prüfkopf von der Oberfläche aus erfolgt, ist der Abstand der Querbügel zum Prüfkopf geringer als der Abstand der Spannbewehrung zum Prüfkopf. Infolgedessen sind die Amplituden der Bügelsignale meistens höher als die der etwaigen Bruchsignale.

Bei der Messung im aktiven Feld können Brüche der Spannbewehrung durch die Methode der Messung bei unterschiedlichen Magnetisierfeldstärken erkannt werden. Hierbei wird das Sättigungsverhalten von ferromagnetischen Materialien ausgenutzt; die oberflächennahe Bewehrung kommt eher in die magnetische Sättigung als die tiefergelegene Spannbewehrung.

Die Brucherkennung erfolgt bei dieser Methode somit durch den Signalvergleich (Korrelationsanalyse, gewichtete Signaldifferenz) von Messungen, die bei unterschiedlicher Magnetisierungsfeldstärke erfolgten. Das Meßsignal enthält hier jedoch noch immer die Signale der Querbügel.

Zur Unterdrückung der Bügelsignale bei der Restfeldmessung werden die Querbügel mit Prüfanordnungen und Verfahren des Standes der Technik durch eine nicht näher beschriebene Führung der Magnetisierung in zahlreichen Schritten entmagnetisiert, so daß nur noch die Signale einer Längsbewehrung bzw. Spannbewehrung übrig bleiben. Hierbei besteht jedoch die Möglichkeit, daß die Längsbewehrung ebenfalls zumindest teilweise entmagnetisiert wird, und somit mögliche Bruchsignale nicht erkannt werden (vgl. DE 4037992).

Die Aufgabe der vorliegenden Erfindung besteht daher darin, eine Prüfanordnung und ein Verfahren zu schaffen, mit der/dem die Signale der Querbügel rechnerisch eliminiert werden können, ohne daß eine aktive Entmagnetisierung des Bauteils vorgenommen werden muß.

Die Aufgabe wird dadurch gelöst, daß der Prüfkopf das Bauteil über eine vorbestimmte Meßstrecke in zwei aufeinanderfolgenden Magnetisierungsvorgängen mit entgegengesetzten Polaritäten magnetisiert, und daß die Steuereinrichtung die Magnetisierungseinrichtung nach Abschluß eines Magnetisierungsvorganges mit einer Polarität ausschaltet und die nach Ausschaltung über die Meßstrecke aufgenommenen Signale speichert und verarbeitet.

Die Aufgabe wird zudem auch durch die Merkmale des Anpruchs 8 gelöst.

Der Vorteil der erfindungsgemäßen Prüfanordnung bzw. des erfindungsgemäßen Verfahrens im Vergleich zur aktiven Entmagnetisierung der Querbügel (etwa durch Anlegen von Gegenfeldern, bzw. magnetischen Wechselfeldern) des Standes der Technik besteht darin, daß die Polstärke P der Magnetisiervorrichtung bei der Magnetisierung konstant bleiben kann. Damit entfällt eine aufwendige Steuerung der Polstärke der Magnetisiervorrichtung bei der Entmagnetisierung der Bügel, die u. U. auch die Magnetisierung der Längs- und insbesonders der Spannbewehrung beeinflussen kann. Zum anderen sind im Vergleich zum eingangs beschriebenen Stand der Technik lediglich zwei Magnetisierungsfahrten erforderlich. Damit kann der Untersuchungsaufwand erheblich gesenkt werden.

Ein weiterer Vorteil gemäß Anspruch 2 besteht darin, daß sich der Prüfkopf mit der Magnetisierungseinrichtung längs des zu untersuchenden Bauteils bewegen kann, wobei das Magnetfeld dieser Magnetsierungseinrichtung in erster Näherung durch das Feld eines Jochmagneten beschrieben werden kann.

Ein weiterer Vorteil gemäß Anspruch 3 und 9 besteht darin, daß der Prüfkopf in wenigstens teilweise einander entgegengesetzten Richtungen verfahrbar angeordnet ist, um so in dem Bauteil Magnetisierungen mit unterschiedlicher Polarität zu erzeugen.

Ein weiterer Vorteil gemäß gemäß Anspruch 4 und 13 besteht darin, daß die Steuereinrichtung die Signale der Magnetisierung mit unterschiedlicher Polarität rechnerisch überlagert. Dadurch heben sich die Signale der Querbügel auf.

Weitere Vorteile der vorliegenden Erfindung ergeben sich aus den Merkmalen der weiteren Unteransprüche 5-7 und 10-12 sowie 14 bis 16.

Eine Ausführungsform der vorliegenden Erfindung wir im folgenden anhand der Zeichnungen näher beschrieben. Es zeigen:

Fig. 1a      eine schematische Vorderansicht eines typischen Spannbetonträgers;

Fig. 1b      eine schematische Seitenansicht des Spannbeton trägers aus Fig. 1a;

Fig. 2      eine schematische Ansicht einer Prüfanordnung mit Prüfkopf zur magnetischen Streufeldmessung gemäß vorliegender Erfindung;

Fig. 3      Diagramm des Magnetfeldes $\underline{H}O$ des Prüfkopfes im Abstand y=10cm von der Mitte des Prüfkopfes;

Fig. 4      Diagramm des Streufeldverlaufs eines Querbügels, Messung bei Hinfahrt im aktiven Feld;

Fig. 5      Diagramm des Streufeldverlaufs eines Querbügels einer ersten Restfeldmessung R1 nach Ausschalten der Magnetisierungseinrichtung an einem Endpunkt einer vorbestimmten Meßstrecke;

Fig. 6      Diagramm des Streufeldverlaufs eines Querbügels einer zweiten Restfeldmessung R2 nach Ausschalten der Magnetisierungseinrichtung am Startpunkt der Meßstrecke aus Fig. 5;

Fig. 7      Diagramm eines gemittelten signalverlaufs der Restfeldmessungen R1 und R2 aus Fig. 5 und Fig. 6;

Fig. 8      schematisches Ablaufdiagramm des Verfahrens gemäß vorliegender Erfindung;

Fig. 9      Diagramm einer ersten Restfeldmessung R1 eines Beispiels unter Verwendung der Prüfanordnung und des Verfahrens gemäß vorliegender Erfindung;

Fig.10      Diagramm einer zweiten Restfeldmessung R2 des Beispiels aus Fig. 9;

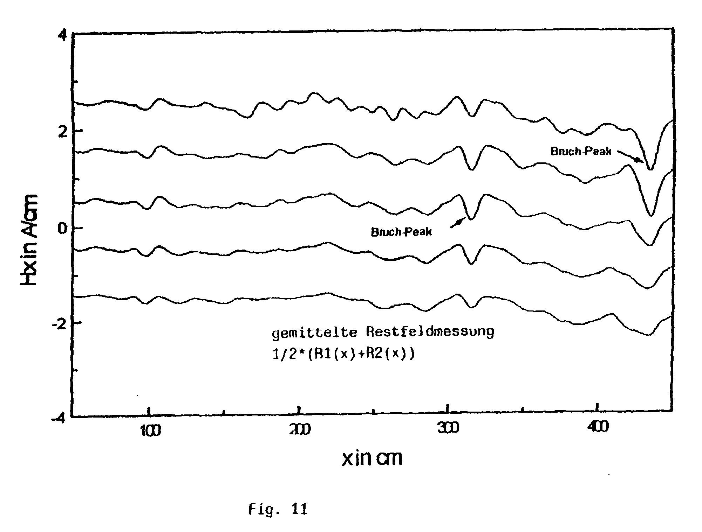

Fig. 11      Diagramm eines gemittelten Signalverlaufs der Restfeldmessungen R1 und R2 aus Fig. 9 und Fig. 10.

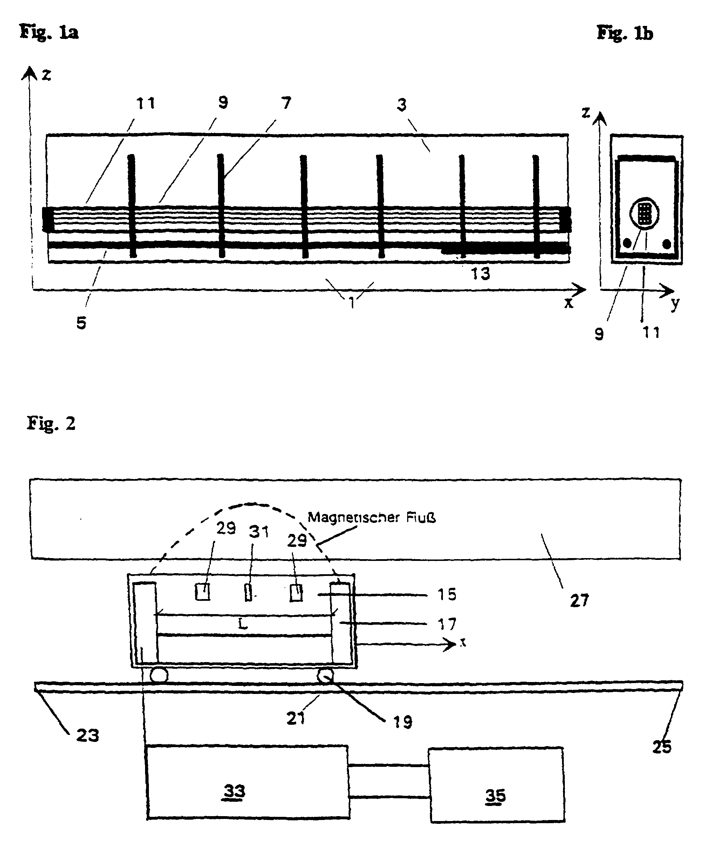

In Fig. 1a ist der typische Aufbau eines Spannbetonträgers 1 in Vorderansicht dargestellt. Im Beton 3 ist eine Bewehrung eingebettet, die im einzelnen zusammengesetzt ist aus Längsbewehrung 5, Querbügel 7, Hüllrohr 9 mit Spannbewehrung 11 und einem Übergreifungsstoß 13. In Fig. 1b ist der Spannbetonträger 1 aus Fig. 1a in einer Seitenansicht dargestellt, um die an sich bekannte räumliche Anordnung wiederzugeben. Der Aufbau eines solchen Spannbetonträgers ist allgemein bekannt und soll daher hier nicht weiter beschrieben werden.

In Fig. 2 ist die erindungsgemäße Prüfanordnung schematisch dargestellt. Ein Prüfkopf 15 umfaßt eine Magnetisierungseinrichtung 17, die im vorliegenden Fall ein Elektromagnet mit einer Jochlänge L ist. Der Prüfkopf 15 ist auf Rollen 19 auf einer schienenförmig ausgebildeten Meßstrecke 21 abgestützt. Die Länge x der Meßstrecke 21 ist vorbestimmt. In Fig. 2 befindet sich ein Startpunkt 23 am linken Ende der Meßstrecke 21 und eine Endpunkt 25 am rechten Ende. Der Prüfkopf 15 ist unterhalb eines zu prüfenden Bauteils 27 angeordnet und ausgerichtet. Vorzugsweise verläuft die Bewegungsbahn des Prüfkopfes 15 oberflächenparallel zu der Unterseite des Bauteils 27.

Im oberen Bereich des Prüfkopfes 15 sind zwei Pick-Up- Spulen 29 und ein Magnetfeld-Sensor 31 angeordnet.

Der Prüfkopf 15 ist mit einer Steuereinrichtung 33 wirkverbunden. In der vorliegenden Ausführungsform ist die Steuereinrichtung 33 mit dem Prüfkopf 15 über eine Leitung extern verbunden. Es ist aber auch denkbar, die Steuereinrichtung 33 in den Prüfkopf 15 zu integrieren. Die Steuereinrichtung 33 ist so ausgebildet, daß sie sowohl den Antrieb des Prüfkopfes 15 als auch die Ein- und Ausschaltung der Magnetisierungseinrichtung 17 durchführt. Die Steuereinrichtung 33 ist zudem auch mit einer Signalverarbeitungseinheit 35 verbunden. In einem Speicherbereich 37 der Signalverarbeitungseinheit 35 werden die Signalverläufe einzelner Magnetfelder und im vorliegenden Fall insbesondere der Restfelder gespeichert und bedarfsweise zur Weiterverarbeitung abgerufen.

Der magnetische Fluß durch das Bauteil ist in Fig. 2 mit einer punktierten Linie angedeutet. Das Magnetfeld der Magnetisierungseinrichtung 17 wird an einem durch die Koordinaten x (Länge), y (Breite) und z (Höhe) bestimmten Ort durch folgende Beziehungen beschrieben:

$$\underline{H}_0 = -P \cdot \mathbf{grad} \left[ \frac{1}{r_1} - \frac{1}{r_2} \right]$$

$$r_{1,2} = \sqrt{(x_h \, m \, L / 2 - x)^2 + y^2 + z^2} \qquad (1)$$

P ist die Polstärke, L ist die Jochlänge und $x_h$ ist hierbei die Position der Mitte des Jochmagneten bzw. der Magnetisierungeinrichtung 17. Es wird weiter in G1.(1) vorausgesetzt, daß die Pole des Jochmagneten die Koordinaten $z_h = y_h = 0$ haben.

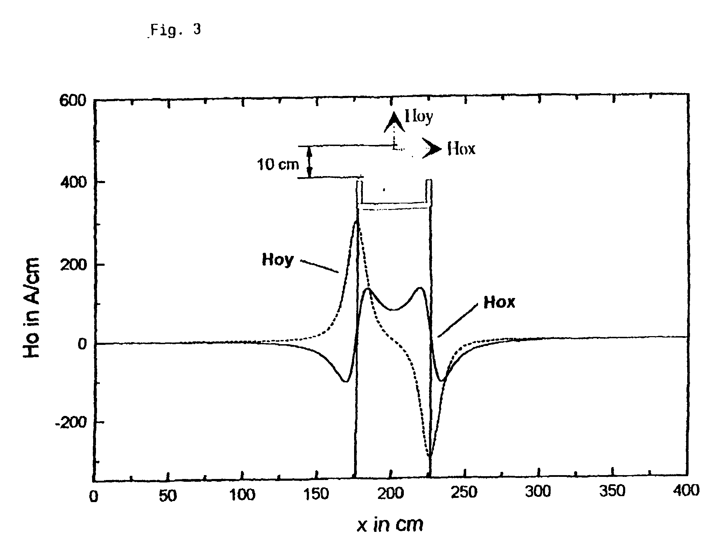

In Fig. 3 sind die senkrechte y- und die axiale x- Komponente des Magnetfeldes dargestellt, wobei die Mitte des Joches bei x = 200 cm angeordnet ist und der senkrechte Abstand zur Polebene 10 cm beträgt.

Aufgrund des geometrischen Entmagnetisierungsfaktors der als langgestreckte Ellipsoide zu betrachtenden Quer- und Längsbewehrungen 7, 5 kann davon ausgegangen werden, daß die längs zur x-Achse angeordnete Spannbewehrung 11 durch die axiale Feldkomponente Hox, die Querbügel 7 jedoch durch die senkrechte Feldkomponente Hoy magnetisiert werden. Aus Fig. 3 wird ersichtlich, daß die senkrechte Feldkomponente Hoy antisymmetrisch in Bezug auf die Jochmitte ist. Bei der Vorbeifahrt des Prüfkopfes 15 an einem Querbügel 7 wird dieser, hinreichende Magnetisierungsfeldstärke vorausgesetzt, ummagnetisiert. Das Vorzeichen des Restfeldsignals des Querbügels 7 hängt deshalb davon ab, ob bei der vorangegangen Magnetisierung das Magnetfeld des Prüfkopfes 15 lediglich bei der Hinfahrt eingeschaltet war und am Endpunkt 25 der Messtrecke 21 ausgeschaltet wird (Fall R1), oder ob das Magnetfeld während der Hin- und Rückfahrt eingeschaltet ist und das Ausschalten der Magnetisiervorrichtung 17 am Startpunkt 23 der Messung erfolgt (Fall R2).

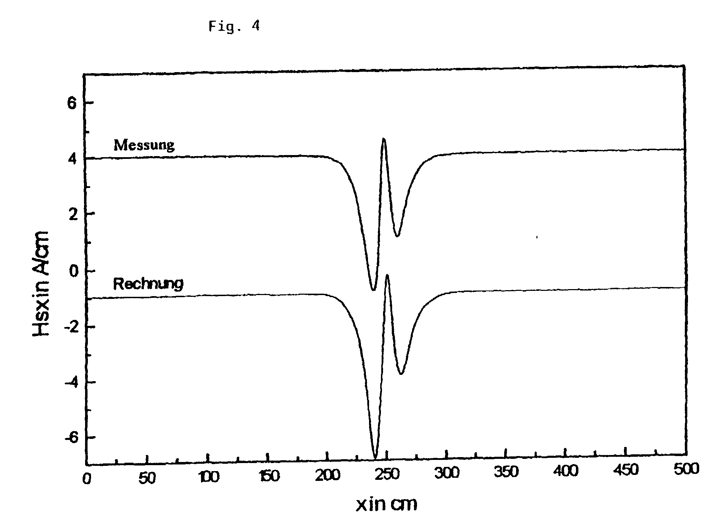

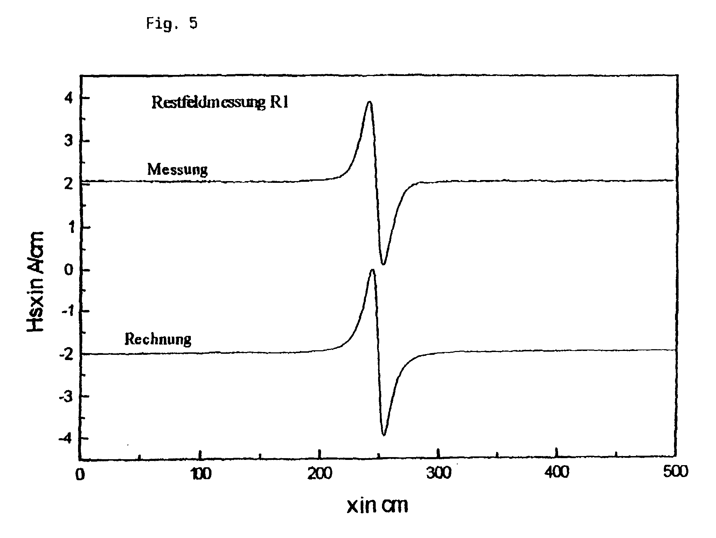

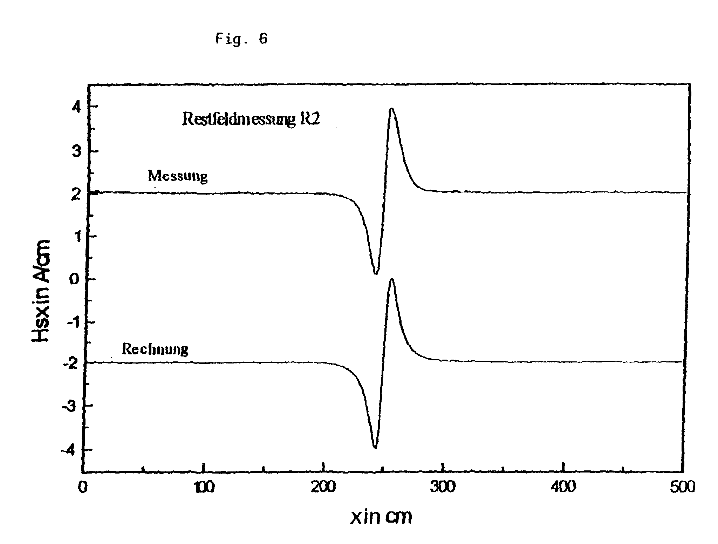

In den Figuren 4-7 sind hierzu berechnete und gemessene Streufeldverläufe (x-Komponente) aufgetragen, die von einem mittig im Prüfkopf 15 angeordneten Sensor 31 bei der Vorbeifahrt an einem Querbügel 7 gemessen werden. Die Rechnungen wurden mit einem nichtlinearen Programm zur Simulation von Streufeldmessungen vorgenommen. Bei den Messungen wurden folgende Randbedingungen gewählt:

| Meßstrecke 21 | Meßbeginn bei xo = 0 cm;<br>Meßende bei x1 = 500 cm, |
|---|---|
| Magnetisierungseinrichtung 17 | Polstärke: P = 75000 Acm,<br>Jochlänge L = 50 cm |
| Bügel 7 (x=250cm) | Durchmesser=1 cm<br>Länge 50 cm<br>Abstand zum Prüfkopf 15, 7,0 cm |

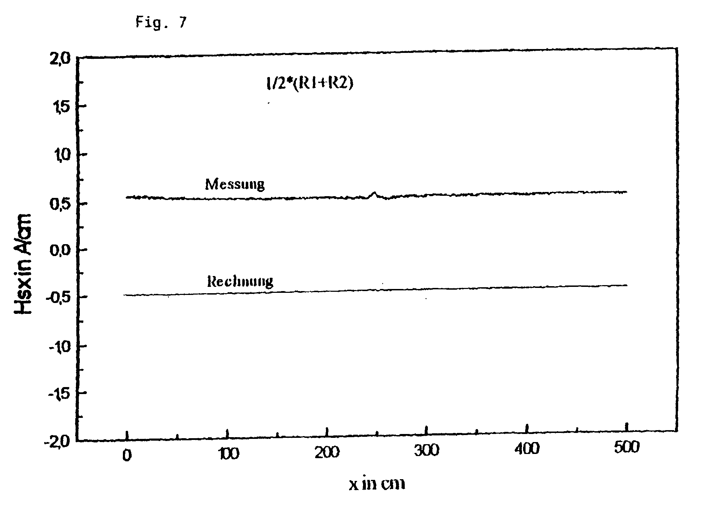

Es wird ersichtlich, daß sich das Signal des Querbügel 7 bei Messung im aktiven Feld erheblich von der Signalform bei der Restfeldmessung unterscheidet; die Amplituden sind jedoch in der gleichen Größenordnung. Bei den Restfeldmessungen R1 und R2 werden gleiche Signalformen, jedoch mit unterschiedlichem Vorzeichen erhalten. Bei der Bildung eines Mittelwertes aus beiden Messungen heben sich die Signale der Querbügel 7 nahezu auf. Dies ist besonders in Fig. 7 zu sehen.

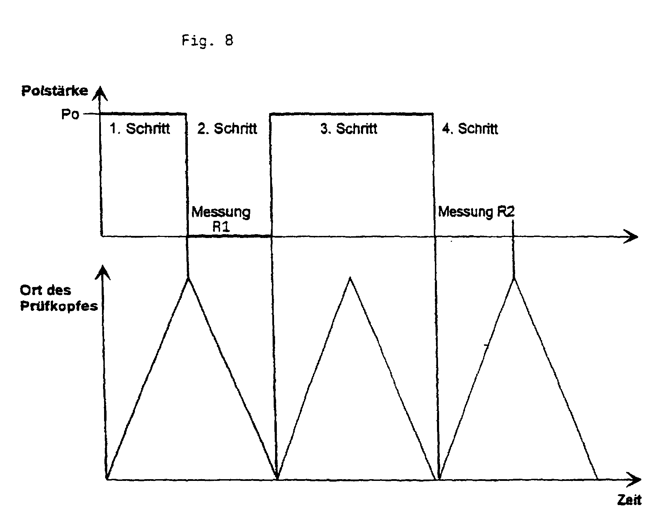

Im folgenden werden der Verfahrensablauf zur Eliminierung der Signale der Querbewehrung gemäß Fig. 8 in seinen wesentlichen Schritten näher beschrieben :

### 1. Schritt: Magnetisierung
Der Prüfkopf 15 wird mit eingeschalteten konstantem Magnetfeld (Polstärke P=Po) vom Startpunkt 23 bis zum Endpunkt 25 der Meßstrecke 21 bewegt und hier (am Endpunkt) ausgeschaltet (Polstärke P=0).

### 2. Schritt : Restfeldmessung R1(x)
Durchführung der Restfeldmessung bei Polstärke P=0 bei der Rückfahrt des Prüfkopfes 15 oder bei einer neuen Hinfahrt des Prüfkopfes 15 zum Endpunkt 25. Speicherung der Meßwerte in Abhängigkeit vom Ort x.

### 3. Schritt: erneute Magnetisierung
Der Prüfkopf 15 wird mit eingeschalteten Magnetfeld (Polstärke P=Po) vom Startpunkt 23 bis zum Endpunkt 25 und wieder zurück zum Startpunkt 23 bewegt und hier (am Startpunkt 23) ausgeschaltet.

### 4. Schritt: Restfeldmessung R2(x)
Durchführung einer erneuten Restfeldmessung bei Polstärke P=0 bei der Rückfahrt oder bei einer neuen Hinfahrt des Prüfkopfes 15. Speicherung der Meßwerte in Abhängigkeit vom Ort x

### 5. Schritt: Rechnerische Mittelung R(x)
Rechnerische Überlagerung durch Addition der beiden Messungen R1 und R2 an jeder Ortskoordinate x der Meßstrecke 21:

$$R(x) = 1/2 \ (R1(x)+ R2(x)) \tag{2}$$

Im Datensatz R(x) sind somit fast nur noch die Signale der längs zur Verfahrrichtung angeordneten Bewehrungen vorhanden.

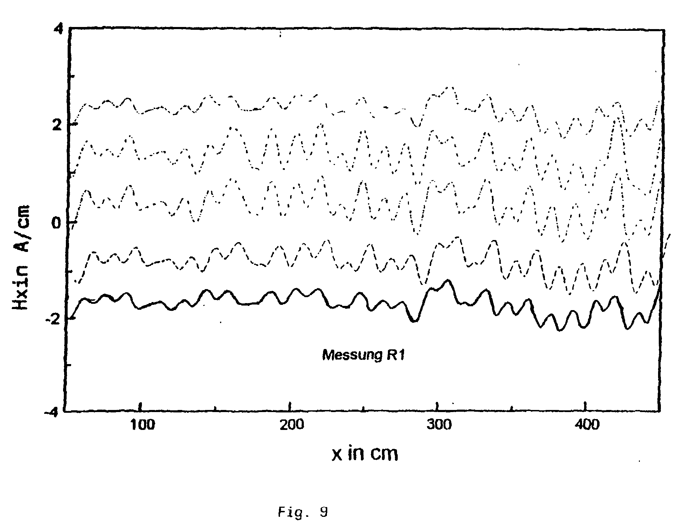

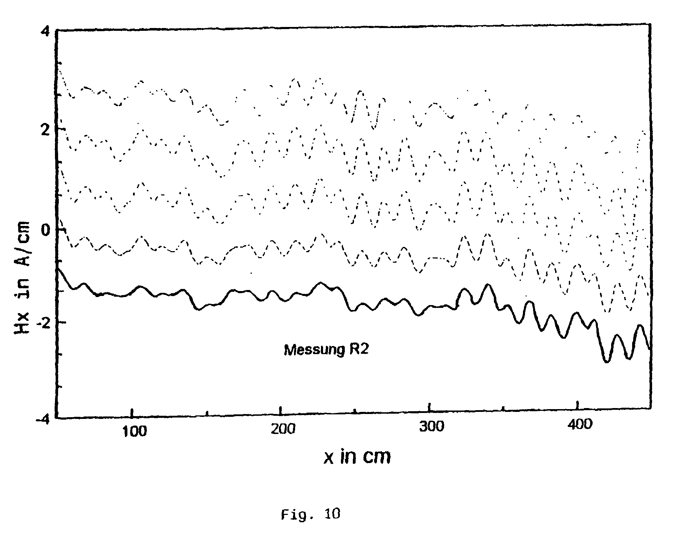

Nachfolgend wird nun das Verfahren an einem praktischen Beispiel demonstriert, bei dem ein Spannbetonträger 1 untersucht wurde. Die Magnetfeldmessung erfolgte hierbei mit 5 Sensoren 31, die im Abstand von 4 cm nebeneinander angeordnet waren. Die Restfeldmessungen Rl und R2 sind in den Figs. 9 und 10 dargestellt:

Aus der gemittelten Restfeldmessung werden zwei Bruchstellen bei x=310 und x=440 cm ersichtlich. Der Bruch bei x=310 liegt hierbei mittig, der Bruch bei 440 cm liegt am Rand des Trägers 1. Das Beispiel zeigt deutlich, daß mit der hier dargestellte Methode bei der Restfeldmessung Bruchsignale der Längsbewehrung 5 gefunden werden können, die in der direkten Restfeldmessung (R1 oder R2) nicht sichtbar sind.

Zusammenfassend sind die wesentlichen Merkmale der Erfindung:

1. Steuerung des Meßablaufs nach dem Ablaufschema nach Bild 8, wodurch zwei Restfeldmessungen durchgeführt werden, bei denen die Magnetisierung aller quer zur Längsbewehrung angeordneten Bewehrungen (Querbügel) entgegengesetzte Polarität haben.

2. Ermittelung des neuen Meßsignals durch Mittelwertbildung nach G1.(2), in dem die Signale der Querbügel weitgehend eliminiert sind, und das im wesentlichen nur noch das Restfeldsignal der längs angeordneten Bewehrungen enthält.

3. Eliminierung der Restfeldsignale der längs angeordneten Bewehrungen durch die Anwendung des Verfahrens in zur ersten Meßrichtung senkrechten Meßrichtungen.

## Patentansprüche

1. Prüfanordnung zur Untersuchung der Integrität von Bauteilen, wie z. B. der Spannbewehrung von Spannbetonbauteilen, mit einem Prüfkopf, der eine Magnetisierungseinrichtung zur Erzeugung eines das Bauteil umgebenden Magnetfeldes aufweist und mit einer Steuereinrichtung zur Steuerung der Magnetisierung und zur Verarbeitung der dem Magnetfeld entsprechenden Signale verbunden ist,
**dadurch gekennzeichnet,**
**daß** der Prüfkopf (15) das Bauteil (27) über eine vorbestimmte Meßstrecke (21) in zwei aufeinanderfolgenden Magnetisierungsvorgängen mit entgegengesetzten Polaritäten magnetisiert, und daß die Steuereinrichtung (33) die Magnetisierungseinrichtung (17) nach Abschluß eines jeden Magnetisierungsvorganges mit einer Polarität ausschaltet und die nach Ausschaltung über die Meßstrecke (21) aufgenommenen Signale speichert und verarbeitet.

2. Prüfanordnung nach Anspruch 1,
**dadurch gekennzeichnet,**
**daß** der Prüfkopf (15) zwischen einem Startpunkt (23) und einem Endpunkt (25) einer vorbestimmten Meßstrecke (21) in entgegengesetzten Richtungen verfahrbar angeordnet ist.

3. Prüfanordnung nach Anspruch 1 oder 2,
**dadurch gekennzeichnet,**
**daß** der Prüfkopf (15) das Bauteil (27) bei den zwei aufeinanderfolgenden Magnetisierungsvorgängen über wenigstens teilweise einander entgegengerichtete Fahrstrecken magnetisiert.

4. Prüfanordnung nach einem der Ansprüche 1 bis 3,
**dadurch gekennzeichnet,**
**daß** die Steuereinrichtung (33) die nach zwei aufeinanderfolgenden Magnetisierungsvorgängen gespeicherten Signale der Magnetisierungen mit unterschiedlicher Polariät rechnerisch überlagert.

5. Prüfanordnung nach Anspruch 3,
**dadurch gekennzeichnet,**
**daß** die Steuereinrichtung (33) die nach zwei aufeinanderfolgenden Magnetisierungsvorgängen gespeicherten Signale der doppelt befahrenen Meßstrecke (21) rechnerisch überlagert.

6. Prüfanordnung nach einem der Ansprüch 1 bis 5,
**dadurch gekennzeichnet,**
**daß** die Meßstrecke (21) zwischen Startpunkt (23) und Endpunkt (25) eine Weglänge x hat und die Magnetisierungseinrichtung (17) bei dem ersten Magnetisierungsvorgang über die Weglänge x und bei dem nachfolgenden zweiten Magnetisierungsvorgang über eine Weglänge 2x eingeschaltet ist.

7. Prüfanordnung nach Anspruch 6,
**dadurch gekennzeichnet,**
**daß** die Steuereinrichtung (33) nach der rechnerischen Überlagerung der Einzelsignale durch Mittelwertbildung ein Ausgangs-Prüfsignal erzeugt.

8. Verfahren zur Untersuchung der Integrität von Bauteilen, wie z. B. der Spannbewehrung von Spannbetonbauteilen, **durch** Magnetisierung des Bauteils, mit den Schritten:

Erzeugen von zwei Magnetisierungen mit unterschiedlicher Polarität in dem Bauteil;
Speichern jeweils der den Magnetisierungen mit unterschiedlicher Polarität entsprechenden Signale; und
Verarbeiten der jeweils den Magnetisierungen mit unterschiedlicher Polarität entsprechenden Signale.

9. Verfahren nach Anspruch 8,
**dadurch gekennzeichnet,**
**daß** der Schritt des Erzeugens von zwei Magnetisierungen mit unterschiedlicher Polarität den Schritt des Bewegens des Prüfkopfes in wenigstens teilweise einander entgegengesetzten Richtungen umfaßt.

10. Verfahren nach Anspruch 8 oder 9,
**dadurch gekennzeichnet,**
**daß** der Schritt des Erzeugens von zwei Magnetisierungen mit unterschiedlicher Polarität die Schritte des Bewegens des Prüfkopfes in einem ersten Magnetisierungsvorgang zwischen einem Startpunkt und einem Endpunkt und in einem zweiten Magnetisierungsvorgang zwischen dem Startpunkt, dem Endpunkt und zurück zum Startpunkt umfaßt.

11. Verfahren nach einem der Ansprüche 8 bis 10,
**dadurch gekennzeichnet,**
**daß** der Schritt des Erzeugens von zwei Magnetisierungen mit unterschiedlicher Polarität den Schritt der Auswahl einer für beide Magnetisierungen gleiche Meßstrecke umfaßt.

12. Verfahren nach Anspruch 11,
**dadurch gekennzeichnet,**
**daß** die Meßstrecken frei festgelegt werden.

13. Verfahren nach einem der Ansprüche 8 bis 12,
**dadurch gekennzeichet,**
daß der Schritt des Verarbeitens der jeweils den Magnetisierungen mit unterschiedlicher Polarität entsprechenden Signale den Schritt des rechnerischen Überlagerns der Signale umfaßt.

14. Verfahren nach Anspruch 13,
**dadurch gekennzeichnet,**
**daß** der Schritt des rechnerischen Überlagerns der Signale eine Mittelwertbildung umfaßt.

15. Verfahren nach einem der Ansprüch 8 bis 14,
**dadurch gekennzeichnet,**
**daß** der Schritt des Speicherns jeweils der den Magnetisierungen mit unterschiedlicher Polarität entsprechenden Signale den Schritt des Bewegens des Prüfkopfes entlang der jeweils vorhergehend magnetisierten Strecke des Bauteils umfaßt.

16. Verfahren nach Anspruch 15,
**dadurch gekennzeichnet,**
**daß** bei dem Schritt des Bewegens des Prüfkopfes entlang der jeweils vorhergehend magnetisierten Strecke des Bauteils den Schritt des Aufnehmens und Speicherns des jeweiligen Restfeldes umfaßt.

**Claims**

1. A test arrangement for examining the integrity of structural components, e.g., the prestressing steel of prestressed concrete parts, with a probe that contains a magnetizing device for generating a magnetic field that surrounds the component, and with a control device for controlling the magnetization and for processing the signals that correspond to the magnetic field, **characterized by** the fact that the probe (15) magnetizes the component (27) with opposite polarities over a predetermined section (21) to be measured in two successive magnetizing processes, and by the fact that the control device (33) switches off the magnetizing device (17) after the completion of each magnetizing process with one polarity, with the control device storing and processing the signals recorded over the section (21) to be measured after switching off the magnetizing device.

2. A test arrangement according to claim 1, **characterized by** the fact that the probe (15) is arranged such that it can be displaced in opposite directions between a starting point (23) and an end point (25) of a predetermined section (21) to be measured.

3. A test arrangement according to claim 1 or 2, **characterized by** the fact that the probe (15) magnetizes the component (27) over sections that at least partially extend opposite to one another during the two successive magnetizing processes.

4. A test arrangement according to one of claims 1-3, **characterized by** the fact that the control device (33) computationally superimposes the stored signals of the magnetizations with different polarities after two successive magnetizing processes.

5. A test arrangement according to claim 3, **characterized by** the fact that the control device (33) computationally superimposes the stored signals of the twice-traveled section to be measured (21) after two successive magnetizing processes.

6. A test arrangement according to one of claims 1-5, **characterized by** the fact that the section (21) to be measured has a length x between the starting point (23) and the end point (25), and by the fact that the magnetizing device (17) is switched on over the length x during the first magnetizing process and over a length 2x during the ensuing second magnetizing process.

7. A test arrangement according to claim 6, **characterized by** the fact that the control device (33) generates a test output signal by means of averaging after the computational superposition of the individual signals.

8. A method for examining the integrity of structural components, e.g., the prestressing steel of prestressed concrete parts, by magnetizing the component, wherein the following steps are carried out:

   generating two magnetizations with different polarities in the component;

   respectively storing the signals that correspond to the magnetizations with different polarities, and

   processing the signals that respectively correspond to the magnetizations with different polarities.

9. A method according to claim 8, **characterized by** the fact that the probe is moved in directions that at least partially extend opposite to one another in the step in which two magnetizations with different polarities are generated.

10. A method according to claim 8 or 9, **characterized by** the fact that the probe is moved between a starting point and an end point during a first magnetizing process and from the start point to the end point and back to the start point during a second magnetizing process, namely in the step in which two magnetizations with different polarities are generated.

11. A method according to one of claims 8-10, **characterized by** the fact that a section to be measured which is identical for both magnetizations is selected in the step in which two magnetizations with different polarities are generated.

12. A method according to claim 11, **characterized by** the fact that the sections to be measured are freely determined.

13. A method according to one of claims 8-12, **characterized by** the fact that the signals are computationally superimposed in the step in which the signals that respectively correspond to the magnetizations with different polarities are processed.

14. A method according to claim 13, **characterized by** the fact that an averaging is carried out in the step in which the signals are computationally superimposed.

**15.** A method according to one of claims 8-14, **characterized by** the fact that the probe is moved along the respective section of the component which was magnetized previously in the step in which the signals that respectively correspond to the magnetizations with different polarities are stored.

**16.** A method according to claim 15, **characterized by** the fact that the respective residual field is recorded and stored in the step in which the probe is moved along the respective section of the component which was magnetized previously.

**Revendications**

**1.** Installation de contrôle pour l'étude de l'intégrité de pièces de construction, comme par exemple l'armature de pièces de construction en béton précontraint, avec une tête de contrôle qui présente un dispositif de magnétisation pour la production d'un champ magnétique entourant la pièce de construction et qui est relié avec une installation de commande pour la commande de la magnétisation et pour le traitement des signaux correspondant au champ magnétique,
**caractérisée en ce que**
la tête de contrôle (15) magnétise la pièce de construction (27) sur une veine de mesure prédéfinie (21) en deux opérations successives de magnétisation avec des polarités opposées, et que l'installation de commande (33) déconnecte le dispositif de magnétisation (17) après l'achèvement de chaque opération de magnétisation avec une polarité et enregistre et, après déconnexion, traite les signaux récupérés sur la veine de mesure (21).

**2.** Installation de contrôle selon la revendication 1,
**caractérisée en ce que**
la tête de contrôle (15) peut être déplacée entre un point de départ (23) et un point final (25) d'une veine de mesure prédéfinie (21) dans des sens opposés.

**3.** Installation de contrôle selon la revendication 1 ou 2,
**caractérisée en ce que**
la tête de contrôle (15) magnétise la pièce de construction (27), lors des deux opérations successives de magnétisation, sur des trajectoires au moins partiellement inverses.

**4.** Installation de contrôle selon une des revendications 1 à 3,
**caractérisée en ce que**
l'installation de commande (33) superpose numériquement les signaux des magnétisations à polarités différentes enregistrés après deux opérations successives de magnétisation.

**5.** Installation de contrôle selon la revendication 3,
**caractérisée en ce que**
l'installation de commande (33) superpose numériquement les signaux de la veine de mesure parcourue deux fois (21) enregistrés après deux opérations successives de magnétisation.

**6.** Installation de contrôle selon une des revendications 1 à 5,
**caractérisée en ce que**
la veine de mesure (21) entre le point de départ (23) et le point final (25) a une longueur de course x et que l'installation de magnétisation (17), lors de la première opération de magnétisation, est réglée sur la longueur de course x et lors de la deuxième opération suivante de magnétisation, sur une longueur de course 2x.

**7.** Installation de contrôle selon selon la revendication 6,
**caractérisée en ce que**
l'installation de commande (33), après la superposition numérique des signaux isolés, produit un signal de contrôle de sortie par formation de valeurs moyennes.

**8.** Procédé pour l'étude de l'intégrité de pièces de construction, comme par exemple l'armature de pièces de construction en béton précontraint, par magnétisation de la pièce de construction, avec les étapes de :

Réalisation de deux magnétisations avec une polarité différente dans la pièce de construction;

Enregistrement respectif des signaux correspondant aux magnétisations à polarité différente;

Traitement des signaux correspondant aux magnétisations à polarité différente.

9. Procédé selon la revendication 8,
**caractérisé en ce que**
l'étape de réalisation de deux magnétisations à polarité différente englobe l'étape de déplacement de la tête de contrôle dans des sens au moins partiellement inverses.

10. Procédé selon la revendication 8 ou 9,
**caractérisé en ce que**
l'étape de réalisation de deux magnétisations à polarité différente englobe les étapes de déplacement de la tête de contrôle, dans une première opération de magnétisation, entre un point de départ et un point final et, dans une deuxième opération de magnétisation, entre le point de départ, le point final et son retour au point de départ.

11. Procédé selon une des revendications 8 à 10,
**caractérisé en ce que**
l'étape de réalisation de deux magnétisations à polarité différente englobe l'étape du choix d'une même veine de mesure pour les deux magnétisations.

12. Procédé selon la revendication 11,
**caractérisé en ce que**
les veines de mesure sont déterminées librement.

13. Procédé selon une des revendications 8 à 12,
**caractérisé en ce que**
l'étape de traitement des signaux correspondant respectivement aux magnétisations à polarité différente englobe l'étape de superposition numérique des signaux.

14. Procédé selon la revendication 13,
**caractérisé en ce que**
l'étape de superposition numérique des signaux englobe une formation de valeurs moyennes.

15. Procédé selon une des revendications 8 à 14,
**caractérisé en ce que**
l'étape de l'enregistrement des signaux respectifs correspondant aux magnétisations à polarité différente englobe l'étape de déplacement de la tête de contrôle le long de la veine respective précédemment magnétisée de la pièce de construction.

16. Procédé selon la revendication 15,
**caractérisé en ce que**
l'étape du déplacement de la tête de contrôle le long de la veine respectivement magnétisée précédemment englobe l'étape de récupération et d'enregistrement du champ résiduel correspondant.

Fig. 1a

Fig. 1b

Fig. 2

Magnetischer Fluß

EP 0 916 089 B1

Fig. 3

Fig. 4

Fig. 5

EP 0 916 089 B1

Fig. 6

EP 0 916 089 B1

Fig. 7

EP 0 916 089 B1

Fig. 8

EP 0 916 089 B1

Fig. 9

Fig. 10

Fig. 11